Europäisches Patentamt

European Patent Office

Office européen des brevets

(19)

(11) Veröffentlichungsnummer: **0 185 372**
**B1**

(12) # EUROPÄISCHE PATENTSCHRIFT

(45) Veröffentlichungstag der Patentschrift:
**01.03.89**

(21) Anmeldenummer: **85116212.3**

(22) Anmeldetag: **19.12.85**

(51) Int. Cl.⁴: **G 01 N 33/543,** G 01 N 33/544,
G 01 N 33/545

(54) Verfahren zur Herstellung eines immunreaktiven porösen Trägermaterials.

(30) Priorität: **20.12.84 DE 3446636**

(43) Veröffentlichungstag der Anmeldung:
**25.06.86 Patentblatt 86/26**

(45) Bekanntmachung des Hinweises auf die Patenterteilung:
**01.03.89 Patentblatt 89/9**

(84) Benannte Vertragsstaaten:
**AT BE CH DE FR GB IT LI LU NL SE**

(56) Entgegenhaltungen:
**EP-A- 0 066 648**

**S.N. TIMASHEFF, G.D. FASMAN "Structure and Stability of Biological Macromolecules", 1969, MARCEL DEKKER, INC., New York**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Schäfer, Rainer, Dr. rer. nat., Tiefentalweg 29, D-8124 Seeshaupt (DE)**
Erfinder: **Jering, Helmut, Dr. rer. nat., Anton-Bartl-Strasse 4, D-8132 Tutzing (DE)**
Erfinder: **Mangold Dieter, DR., Fröhlichstr.62, 6800 MANNHEIM 1 (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

## Beschreibung

Vorliegende Erfindung bietet ein Verfahren zur Herstellung eines immunreaktiven porösen Trägermaterials an.

Immunreaktive poröse Trägermaterialien spielen in verschiedenen Zweigen der Technik eine wichtige Rolle. Beispielsweise werden derartige Materialien für analytische und präparative Verfahren benötigt, bei welchen ein Partner einer Immunreaktion an eine unlösliche Matrix fixiert eingesetzt wird. Die Fixierung des Partners der Immunreaktion an den unlöslichen Träger kann durch chemische oder physikalische Kräfte erfolgen. So sind seit langem Methoden zur Herstellung kovalenter Bindungen zwischen einem festen Trägermaterial und einer daran zu bindenden chemischen Substanz, wie insbesondere auch biologisch aktiven Proteinen, bekannt. Ein Nachteil dieser Methoden besteht generell darin, dass sie eine chemische Veränderung am biologisch aktiven Material bewirken, die in vielen Fällen auch eine Veränderung der biologischen Aktivität zur Folge hat. Eine weitere bekannte Methode ist die Einschlusspolymerisation derartiger biologisch aktiver Substanzen. Hier bleibt zwar das Molekül als solches in der Regel unverändert und behält daher auch seine biologische Wirksamkeit unverändert bei. Die Zugänglichkeit für den anderen, in einer flüssigen Phase vorliegenden Partner der Immunreaktion ist jedoch drastisch beschränkt. Daher wurde vielfach auf das dritte bekannte Verfahren zurückgegriffen, welches die Nachteile der kovalenten Bindung und der Einschlusspolymerisation vermeidet, nämlich die adsorptive Fixierung an einem geeigneten Trägermaterial. Diese Methode wiederum hat jedoch den Nachteil, dass die Bindung an den festen Träger wesentlich schwächer ist als bei den beiden zuerst erwähnten Methoden und nur auf bestimmten Kunststoffoberflächen in der Regel eine akzeptable Bindungsstabilität erzielt werden konnte.

Spezielle Probleme ergaben sich hinsichtlich der Haftfestigkeit bei einer adsorptiven Bindung, bei der also sowohl eine kovalente Fixierung, als auch eine Einschlusspolymerisation, vermieden werden soll dann, wenn poröse Trägermaterialien angewendet werden sollen. Aus der US-PS 3 888 629 ist eine Methode bekannt, die dieses Haftfestigkeitsproblem löst, indem im porösen Trägermaterial die Fixierung durch Ablaufenlassen einer Immunreaktion zwischen den beiden Partnern einer Immunreaktion, also zwischen Antikörper und Antigen oder Hapten, durchgeführt wird. Man imprägniert hierzu das poröse Trägermaterial entweder mit einer Lösung des ersten Partners der Immunreaktion und danach mit einer Lösung des zweiten Partners der Immunreaktion, so dass die Immunreaktion selbst im porösen Trägermaterial ablaufen kann unter permanenter Fixierung des gewünschten immunreaktiven Stoffes ohne chemische Veränderung oder Nachteilen hinsichtlich seiner Zugänglichkeit für andere Reaktionspartner (PCT-WO 82/02601). In einer zweiten bekannten Methode werden die beiden Lösungen der jeweiligen Partner der Immunreaktion erst rasch zusammengemischt und dann sofort das poröse Trägermaterial damit imprägniert (US-A-3 888 629).

Diese Methode der Bindung eines immunreaktiven Stoffes auf einem porösen Trägermaterial löst zwar die oben beschriebenen Probleme, weist jedoch einen anderen Nachteil auf, nämlich eine ungleichmässige Verteilung der fixierten immunreaktiven Substanz auf dem Trägermaterial. Dieser Nachteil ist besonders dann gravierend, wenn dieses immunreaktive poröse Trägermaterial z.B. für Zwecke der quantitativen Analyse niedrig konzentrierter Haptene oder Proteine eingesetzt werden soll. Die Forderung nach Stabilität des Antigen-Antikörper-Netzwerkes sowie nach niedrigen Gleichgewichts-Dissoziations-Konzentrationen der immunreaktiven Partner aus den Immunkomplexen relativ zur Konzentration der zu bestimmenden Haptene und Proteine lässt sich nur erreichen, wenn der verwendete Antikörper eine hohe Affinität zum jeweiligen Antigen besitzt. Erfahrungsgemäss setzt die Präzipitatbildung beim Mischen derartig immunreaktiver Partner spontan ein. Bei der Herstellung technischer Mengen eines Immunadsorbers durch Imprägnieren eines porösen Trägermaterials mit kurz zuvor gemischten Lösungen der immunreaktiven Partner kommt es erwartungsgemäss zu zeitlich und räumlich unkontrollierbaren Präzipitatbildung auf dem Trägermaterial.

Die quantitative Dosierung des Immunosorbens erfolgt zweckmässigerweise durch Ausschneiden einer bestimmten Papierfläche bei einem immunreaktiven Papier, durch Auswahl einer bestimmten Anzahl von Kügelchen bei einem kugelförmigen porösen Trägermaterial oder durch Auswiegen einer bestimmten Menge des immunreaktiven Trägermaterials. Bei ungleichmässiger Verteilung des fixierten immunreaktiven Stoffes scheidet eine derartige einfache Dosierungsmethode jedoch aus.

Ein weiterer Nachteil der bekannten Immunpräzipitationstechnik besteht darin, dass sie sehr hochgereinigte Antigene benötigt, was eine Vorreinigung durch Immunosorption und damit eine aufwendige Herstellungsweise nötig macht.

Der vorliegenden Erfindung liegt nunmehr die Aufgabe zugrunde, auf einfache Weise poröse immunreaktive Trägermaterialien herzustellen, welche eine überlegene Homogenität hinsichtlich der Verteilung des immunreaktiven Bestandteils aufweisen und sich leicht und einfach herstellen lassen, insbesondere keine aufwendige Vorreinigung für die Herstellung erfordern.

Gelöst wird diese Aufgabe erfindungsgemäss durch ein Verfahren zur Herstellung eines immunreaktiven, porösen Trägermaterials durch Aufbringung von je einer Lösung eines ersten Partners einer Immunreaktion und eines mit diesem präzipitierenden zweiten Partners einer Immunreaktion, Inkubation des mit den Lösungen getränkten Trägermaterials zur Immunpräzipitation und Trocknen des imprägnierten Trägermaterials, welches dadurch gekennzeichnet ist, dass man eine Lösung beider Partner der Immunreaktion herstellt, welche einen Hemmstoff für die Immunpräzipitation enthält, das Trägermaterial mit dieser Lösung imprägniert und dann die Immunpräzipitation durch Entfernen des Hemmstoffes oder Aufhebung der Hemmwirkung auslöst.

Wesentliches Merkmal der Erfindung ist die Verwendung eines Immunpräzipitations-Hemmstoffes, der reversibel wirksam ist und durch einfache Entfernung oder chemische Veränderung seine Hemmwirkung verliert. In Gegenwart des Hemmstoffes können die Partner der zur Präzipitation führenden Immunreaktion sich völlig homogen verteilen und aufgrund der sehr langsam einsetzenden Fällung erhält man eine völlig gleichmässige Bedeckung des Trägermaterials mit der jeweils gewünschten immunreaktiven Substanz, die auch im grosstechnischen Ansatz zu völlig gleichmässigen und reproduzierbaren Resultaten führt.

Im Gegensatz zu dem bekannten Verfahren des sequentiellen Aufbringens von Antigen und Antikörper (heterogenes Verfahren), wie es in Beispiel 4 beschrieben ist, wird bei dem erfindungsgemässen «homogenen» Verfahren mit einer homogenen Lösung von Antigen und Antikörper getränkt. Dabei werden zwei Ausführungsformen unterschieden: Beim «1-Schritt-Verfahren» wird der Hemmstoff, der in der Tränklösung enthalten ist, während des Herstellungsverfahrens aus der Lösung entfernt oder unwirksam gemacht. Beim «2-Schritt-Verfahren» wird der poröse Träger zunächst mit einem Wirkstoff vorgetränkt, der die Wirkung des Hemmstoffs bei der nachfolgenden Tränkung mit den immunreaktiven Komponenten aufhebt.

Eine Möglichkeit zur Entfernung des Hemmstoffes ist ein Waschschritt vor der Trocknung, ebenso kann durch Zusätze zur Waschlösung die Wirkung des Hemmstoffes im porösen Trägermaterial aufgehoben werden.

Der Waschschritt ist jedoch kein notwendiger Verfahrensschritt, so kann beispielsweise die Präzipitation durch Abdampfen eines flüchtigen Hemmstoffes ausgelöst werden.

Als Hemmstoffe der Immunpräzipitation werden im Rahmen der Erfindung vorzugsweise solche Substanzen verwendet, welche bei immunosorptiven Aufreinigungen als Desorptionsmittel eingesetzt werden. Besonders bevorzugt werden hierzu Säuren, Basen oder chaotrope Ionen der Hofmeister-Reihe (lyotrope Reihe), wie sie beispielsweise beschrieben sind in «Structure and Stability of Biological Macromolecules», 1969, Marcel Dekker, Inc., New York, Seite 427, sowie bestimmte organische Verbindungen oder Lösungsmittel wie Acetonitril, Harnstoff oder Glycerin.

Unter den im Rahmen des Verfahrens der Erfindung geeigneten Säuren kommen sowohl flüchtige, als auch nicht flüchtige Säuren in betracht. Flüchtige Säuren können nach Imprägnierung des Trägermaterials zur Aufhebung der Hemmwirkung leicht entfernt werden, z.B. durch Erwärmen, Vakuum und dergleichen. Bei nicht flüchtigen Säuren lässt sich ein analoger Effekt durch Einsatz eines Salzes einer flüchtigen Säure, welches von der nicht flüchtigen Säure zerlegt wird, unter Freisetzung der flüchtigen Säure, erzielen. Bevorzugte Beispiele sind Essigsäure, Propionsäure und Salzsäure für flüchtige Säuren. Ebenso lassen sich flüchtige und nicht flüchtige Basen wie z.B. Ammoniak und t-Phosphat verwenden.

Weiterhin sind als Hemmstoffe organische Verbindungen geeignet, welche reversibel sowohl die Protein- als auch die Wasserstruktur beeinflussen können und beispielsweise in «J.F. Brandts, Conformatial Transitions of Proteins in Water», enthalten in «Structure and Stability of Biological Macromolecules, 1969, Marcel Dekker, Inc., New York, Seite 213–290 beschrieben sind. Besonders bevorzugt werden Glycerin und Harnstoff.

Als Hemmstoffe kommen weiterhin in Frage chaotrope Ionen, wie Thiocyanate und Jodide. Beispiele für andere, geeignete Ionen sind: Fluoride, Bromide, Perchlorate, Guanidin und Sulfate. Ihre Entfernung zwecks Aufhebung der Präzipitationshemmung kann durch Extraktion, z.B. mit organischen Lösungsmitteln oder Gemischen von organischen Lösungsmitteln (z.B. Ester) oder Gemischen aus organischen Lösungsmitteln und Wasser, beispielsweise Wasser/Alkohol-Gemischen gegebenenfalls mit Zusatz von Ionophoren und dergleichen erfolgen. In der Regel genügt dabei bereits eine Veränderung der Ionenstärke, um den gewünschten Effekt zu erzielen, doch kann auch eine vollständige Entfernung des Hemmstoffes erfolgen. Auch ein Zusatz von Komplexbildnern wie EDTA kommt in Frage, z.B. zur Entfernung von hemmenden Metallsalzen wie $MgCl_2$.

Die molaren Konzentrationen der zur Präzipitation führenden immunreaktiven Partner können in weiten Bereichen variiert werden. Vorzugsweise werden sie bei der Erfindung so eingesetzt, dass derjenige Partner, dessen Immunreaktivität im fertigen Trägermaterial ausgenützt werden soll, nicht im Überschuss vorliegt. Besonders bevorzugt werden die zur Präzipitation führenden Partner im Verhältnis des Heidelberger Maximums eingesetzt. Dies heisst, dass die beiden Reaktionspartner in dem für die Präzipitation günstigsten Verhältnis eingesetzt werden. Dieses Verhältnis lässt sich durch Trübungstests leicht vorher festlegen. Eine entsprechende Trübungskurve findet sich beispielsweise in «Methods in Immunology and Immunochemistry», Band III, Academic Press 1971, Kapitel 13, Seite 10. Bei der Erstellung einer solchen Kurve wird die Trübung aufgetragen, die bei konstanter Menge eines der Reaktionspartner mit zunehmenden Mengen des anderen Reaktionspartners erhalten wird. Das Trübungsmaximum ist das Heidelberger Maximum.

Um möglichst hohe Stabilität des trägerfixierten immunreaktiven Körpers zu erreichen, soll einer der Partner der Immunpräzipitation vorzugsweise ein präzipitierender Antikörper mit möglichst hoher Affinität zum Antigen sein.

Die Partner der Immunpräzipitation im Rahmen des erfindungsgemässen Verfahrens sind einerseits ein Antigen z.B. ein Hapten oder ein Protein, andererseits ein Antikörper. Das Antigen kann natürlich selbst ebenfalls einen Antikörper darstellen. In einem solchen Falle, wenn also der zu präzipitierende immunreaktive Bestandteil selbst ein Antikörper ist, kann dieser auch als Fab, Fab' bzw. (Fab')$_2$-Fragment eingesetzt werden und zwar sowohl eines monoklonalen, als auch eines polyklonalen Antikörpers. Der präzipitierende Anti-Antikörper, der dann für die Fällung eingesetzt wird, muss entsprechend ausgewählt werden. Der Anti-Antikörper selbst kann dann polyklonal oder monoklonal oder ein (Fab')$_2$-Fragment davon sein. Handelt es sich beim Anti-Antikörper um einen monoklonalen Antikörper oder ein Fragment davon, so sollten diese zweckmässig zwei unterschiedliche Epitope am Antigen erkennen. Jedoch können auch monoklonale Antikörper bzw. deren (Fab')$_2$-Fragmente verwendet werden, die nur ein Epitop erkennen (was häufiger ist), wenn dieses Epitop auf dem Antigen mindestens zweimal vorhanden ist. Auch können Mischungen von monoklonalen Antikörpern als Anti-Antikörper-Fraktion für die Immunpräzipitation eingesetzt werden.

Als zu präzipitierender immunreaktiver Stoff kann vorzugsweise auch ein Protein verwendet werden, an das ein Hapten oder Antigen gekoppelt ist. In diesem Falle wird zweckmässig ein präzipitierender Antikörper als zweiter Partner der Immunreaktion verwendet, welcher gegen das Protein gerichtet ist. In einer weiteren Ausführungsform kann an den präzipitierenden Antikörper ein Hapten oder Antigen gekoppelt sein. Dabei kann der andere Partner der Immunrekation unmarkiert oder mit dem gleichen oder einem anderen Hapten oder Antikörper gekoppelt sein. Alternativ ist das zu präzipitierende Protein selbst ein spezifischer Antikörper, der von einem Anti-Antikörper präzipitiert wird.

Wie oben bereits erwähnt, ist es ein spezieller Vorteil des erfindungsgemässen Verfahrens, dass man wenigstens einen Partner der Immunreaktion in ungereinigter Form einsetzen kann. Im Gegensatz zu den bekannten Immunpräzipitations-Verfahren, bei denen die Präzipitation augenblicklich nach Zusammengeben der Immunreaktionspartner einsetzt, kann bei dem erfindungsgemässen Verfahren die Geschwindigkeit der Präzipitation in Suspension oder im porösen Trägermaterial so kontrolliert erfolgen, dass keine Mitreisseffekte bei Fremdsubstanzen auftreten und letztere nicht vorher entfernt werden müssen, sondern nach der Immunpräzipitation einfach ausgewaschen werden können. Ein derartiger Waschschritt ist bevorzugt, da hierbei kleine Präzipitatpartikel, weniger stabile Immunkomplexe und adsorptiv gebundene Antigene entfernt werden. Zur Waschung eignen sich daher Puffer mit hoher Ionenstärke, Detergenzien, organische Lösungsmittel

und dergleichen sowie die Präzipitate stabilisierende Zusätze (Kohlenhydrate und Proteine).

Wenn auf eine Vorreinigung der Komponenten der Immunpräzipitationsreinigung verzichtet werden soll, ist es zweckmässig, Lösungen, welche das jeweilige Antigen und den jeweiligen Antikörper enthalten, erst im optimalen (Heidelberger-)Verhältnis zu mischen, das ausgefallene Präzipitat zu waschen und durch Zusatz des Hemmstoffes der Immunpräzipitation, vorzugsweise durch Säurezusatz, wieder aufzulösen. Man erhält so eine Mischung der Partner der Immunpräzipitationsreaktion, die direkt auf das poröse Trägermaterial aufgetragen werden kann.

Natürlich ist es im Rahmen der Erfindung auch möglich, die Partner der Immunreaktion vorher getrennt über Immunadsorber in üblicher Weise aufzureinigen, dann mit einem Hemmstoff zu eluieren und die Eluate entweder direkt für das erfindungsgemässe Verfahren einzusetzen oder erst durch Lyophilisation stabile Lagerformen der Reaktionspartner herzustellen. Die Hemmung der Immunpräzipitation sowie die Verlangsamung der Fällung im Trägermaterial nach Aufhebung der Hemmwirkung kann durch physikalische Methoden unterstützt werden, wie z.B. Temperaturerniedrigung oder Viskositätserhöhung.

Als Träger kommen im Rahmen der Erfindung übliche feste Träger für immunreaktive Substanzen in betracht. Ein derartiges Trägermaterial, welches häufig auch als Matrix bezeichnet wird, kann beispielsweise aus Glas, Kunststoff, Papier, porösem Metall und dergleichen bestehen, vorausgesetzt, das Trägermaterial ist ausreichend von zusammenhängenden flüssigkeitsdurchlässigen Hohlräumen durchsetzt. Geeignet sind natürliche, künstliche, organische oder anorganische Polymere. Ebenso kommen faserartige, schwammartige oder gesinterte Substanzen in betracht. Als Trägermaterialien können flächige, partikuläre oder andere dreidimensionale poröse Körper eingesetzt werden. Bevorzugt werden flächige poröse Träger verwendet wie Papier, Schaumstoff-Folien, Glasfasermatten und dergleichen. Da das erfindungsgemässe Verfahren eine völlig homogene Verteilung der immunreaktiven Substanz in diesen flächigen Matrizen ergibt, kann eine Dosierung höchst einfach anhand der Flächeneinheiten erfolgen. Derartige immunreaktive flächige Trägermaterialien eignen sich besonders für Verwendung als feste Phase in den heterogenen Immunoassays.

Die Anwendung eines erfindungsgemäss hergestellten immunreaktiven Trägermaterials im Rahmen des heterogenen Enzymimmunoassays kann z.B. so erfolgen, dass Hapten (H) oder Protein (P), das in einer Probe wie Pufferlösung, Serum, Plasma, Urin, Kulturüberstand, u.a. enthalten ist, mit einem markierten Binder (B) versetzt wird. Als Binder kommen u.a. Antikörper, Fab$^-$-Fragment bzw. Fab-Fragment sowie Liganden in Frage, welche spezifisch mit dem Hapten oder Protein reagieren. Als Markierung des Binders kann z.B. ein Enzym, Fluoreszens-Label oder Radioisotop, verwendet werden, in den nachfolgenden Beispielen wurde die β-Galactosidase gewählt. Die Menge des zugegebenen Binders kann molmässig sowohl im Überschuss als auch im Unterschuss zu dem in der Probe vorhandenen Hapten oder Protein vorliegen.

Diese Mischung wird für eine konstante Zeit inkubiert, während der sich die Komplexe H–B bzw. P–B bilden. Nach Ablauf dieser Zeitspanne liegen im Reaktionsgemisch drei Spezies vor, nämlich der Komplex, bestehend aus Hapten/Protein, und Binder (H–B, P–B), restliches freies Hapten/Protein (H/P) und restlicher freier Binder (B).

Die Trennung dieser Spezies erfolgt in einem zweiten Schritt mittels Immunosorption. Dazu wird das Gemisch auf die erfindungsgemäss hergestellte Immunpräzipitation-Festphase aufgetragen, die das nachzuweisende Hapten bzw. den Antikörper gegen das zu bestimmende Protein gebunden enthält.

- Beim Hapten-Test bindet nunmehr der freie Binder, nicht aber der mit Hapten abgesättigte Binder an die Festphase. Folglich enthält der Überstand bzw. das Eluat der Festphase den mit dem Hapten der Probe abgesättigten Binder. Die quantitative Bestimmung des Haptens erfolgt nunmehr über die Markierung des Binders, in den vorliegenden Beispielen über die Bestimmung der β-Galactosidase mittels o-Nitrophenyl-β-D-galactosid oder Chlorphenolrot-β-D-galactosid.

- Beim Protein-Test bindet der Komplex aus Protein und Binder, nicht jedoch der freie Binder an die Festphase, Reste des freien Binders werden durch Waschen vom erfindungsgemäss hergestellten Immunosorbens entfernt. Der quantitative Nachweis des Proteins erfolgt über den markierten Binder, in den folgenden Beispielen über die Bestimmung der β-Galactosidase mittels o-Nitrophenyl-β-D-galactosid oder Chlorphenolrot-β-D-galactosid.

Eine andere Anwendung sind kompetitive Immuntests. Der Einsatz des erfindungsgemäss hergestellten immunreaktiven Trägermaterials kann so erfolgen, dass der Analyt (Hapten oder Protein), der in der Probe enthalten ist, mit einer konstanten Menge an markiertem Analyten versetzt wird. Die Markierung kann z.B. ein Enzym, ein Fluoreszenz-Label, Radioisotop und anderes sein. Diese Mischung wird auf die Matrix gegeben. Auf der Matrix ist ein Antikörper immobilisiert, welcher gegen den Analyten gerichtet ist. Die Mischung wird auf der Matrix eine bestimmte Zeit inkubiert. Während dieser Zeit konkurrieren sowohl unveränderter Analyt als auch markierter Analyt um die Bindungsstellen auf der Matrix. Je mehr Analyt in der Probe vorhanden ist, desto weniger markierter Analyt wird von der Matrix gebunden und umgekehrt. Am Ende der Inkubationsphase wird die Flüssigkeit aus der porösen Matrix entfernt, z.B. durch Zentrifugieren. Bestimmt wird dann die Menge des markierten Analyten entweder in der freien Phase oder die Menge des an die Matrix gebundenen markierten Analyten.

Eine weitere Anwendung der erfindungsgemäss hergestellten Trägermaterialien kann so erfolgen, dass der Analyt (Hapten oder Protein) mit einer konstanten Menge an markiertem Antikörper, der gegen den Analyten gerichtet ist, versetzt wird. Mögliche Arten der Markierung des Antikörpers wurden weiter oben beschrieben. Diese Mischung wird entweder eine bestimmte Zeit inkubiert und dann auf das poröse Trägermaterial gegeben oder alternativ sofort nach Mischung auf das poröse Trägermaterial gegeben. Wenn der Analyt ein Hapten ist, enthält das Trägermaterial das nachzuweisende Hapten oder ein Derivat davon in fixierter Form, falls der Analyt ein Protein ist, enthält das Trägermaterial das nachzuweisende Protein oder ein Derivat davon ebenfalls in fixierter Form. Wurde die erste Mischung vor Auftragung auf das Trägermaterial inkubiert, so bindet markierter Antikörper mit noch freien Bindungsstellen an das poröse Trägermaterial. Wurde die Mischung sofort auf das Trägermaterial gegeben, so konkurrieren der Analyt aus der Probe und der an das Trägermaterial fixierte Analyt um die Bindungsstellen des markierten Antikörpers. Am Ende der Inkubationsphase wird die Flüssigkeit aus dem porösen Trägermaterial entfernt und die Menge des markierten Antikörpers entweder in der flüssigen Phase oder auf dem porösen Trägermaterial bestimmt.

Die folgenden Beispiele erläutern die Erfindung weiter.

Gewinnung der für den Test verwendeten Einsatzstoffe und Testführung

A) Herstellung von Polyhaptenen (PH):

Die Herstellung von PH ist Stand der Technik. So kann z.B. ein Digoxin- oder Diphenyl-Hydantoin-PH über reaktive unsymmetrische Dicarbonsäureester/aktivierte Haptenester und deren Bindung an ein Trägerprotein gewonnen werden.

Die Herstellung von T3- und T4-PH kann beispielsweise durch direkte Kupplung der NH$_2$-Gruppen von Hormon und Protein mittels Bis-Imidaten erfolgen (EP-A 0 078 952), oder durch die Carbodiimid-Reaktion (Aherne et al., Brit. J. Clin. Pharm. 3, 56 (1976)) oder die «gemischte-Anhydrid»-Reaktion (Erlanger et al., Methods in Immunology and Immunochemistry, ed. Williams and Chase, Acad. Press (New. York 1967) p. 149 ff). Alternativ kann die NH$_2$-Funktion von T3 oder T4 in einem ersten Schritt mit der Acetyl-, Trifluoracetyl-, t-Butoxicarbonyl- oder der Benzyl-oxicarbonyl-Gruppe geschützt werden. Anschliessend wird die Carboxyl-Funktion in einen aktivierten Ester überführt, z.B. N-hydroxysuccinimidester, N-hydroxyphthalimidester, N-hydroxybenztriazolester. Ein Beispiel für ein so aktiviertes T4 ist beschrieben in EP-A 0 108 400, Beispiel 3. Umsetzung mit dem Trägerprotein ergibt das PH.

Die Auswahl der Trägerproteine unterliegt keinen Einschränkungen, sofern nur ein entsprechender «fällender» Antikörper zur Verfügung steht bzw. hergestellt werden kann.

Soweit eine immunsorptive Aufreinigung von Antigen und Antikörper erfolgte, wurde als Träger das «Affinitätsadsorbens, Glutardialdehyd aktiviert» von Boehringer Mannheim (Best. Nr.

665 525) verwendet. Für die unten beschriebenen Beispiele wurde nach Vorschrift des Herstellers für die Antigen-Aufreinigung ein Schaf-Antikörper gegen Kaninchen-IgG bzw. ein spez. Antikörper gegen das Hapten und für die Antikörper-Aufreinigung Kaninchen-IgG an den Träger gebunden. Die Durchführung der Immunosorption erfolgte, wie in der Arbeitsanleitung zum Affinitätsadsorbens beschrieben.

B) Herstellung des Binders (Beispiel Digoxin):

Gewinnung des Antiserums gerichtet gegen Digoxin. Die Herstellung des Immunogens, nämlich Humanserum-Albumin, konjugiert mit Digoxin, erfolgte so, wie von V.P. Butler jr. und J.P. Chen, Proc. Nat. Acad. Sci, US 51, 71–78 (1967) sowie von T.W. Smith, V.P. Butler und E. Haber, Biochemistry 9, 331–337 (1970) ausführlich beschrieben wurde. Mit diesem Immunogen wurden Schafe immunisiert und das entsprechende Antiserum gewonnen.

Herstellung des Binders

Antiserum, gerichtet gegen Digoxin, wurde über Ammoniumsulfat-Fällung und Passage über DEAE-Cellulose zur IgG-Fraktion aufgereinigt. Die Papain-Spaltung wurde nach R.R. Porter, Biochem. J. 73, 119–126 (1959) durchgeführt.

Die Fab-Fragmente wurden von den nicht verdauten IgG-Molekülen und den Fc-Fragmenten mittels Gelfiltration über Sephadex G 100 und Ionenaustauschchromatographie über DEAE-Cellulose nach Literaturvorschrift (K. Malinowski und W. Manski in «Methods in Enzymology»; J.J. Langone und H. van Vunakis eds., Academic Press, Vol. 73 (1981) pp. 418–459) abgetrennt. Die resultierende Fab-Fraktion wurde chromatographisch gereinigt und an β-Galactosidase nach der Vorschrift von T. Kitiwaga in «Enzyme Immunoassay; Ishikawa, T. Kawai, K. Miyai, eds. Igaku Shoin, Tokyo/New York (1981) pp. 81–89, gekoppelt.

C) Herstellung der Schafantikörper gegen Kaninchen- bzw. Maus-IgG/Fcγ

Kaninchen-Schlachtserum bzw. Mäuseserum wurde einer Ammonsulfat-Fällung unterzogen. Nach Passage über DEAE-Cellulose und Papain-Spaltung, Gel- und Ionenaustauschchromatographie (siehe B) ) erhält man die Fc-Fragmente des Kaninchen- bzw. Maus-IgG's als Immunogene.

Die Aufarbeitung der Schaf-Antiseren erfolgt wie unter B) beschrieben (Immunosorption siehe A)).

D) Testführung (Beispiel Digoxin):

Digoxin-Standards in Humanserum (entnommen aus dem Elisa-Kit von Boehringer Mannheim, Best. Nr. 199 656) wurden 1:7,5 mit 0,9% NaCl-Lösung verdünnt. Zu 200 µl verdünntem Standard wurde 200 µl Binder (20 mU/ml, bestimmt mit ortho-nitrophenyl-β-D-galactosid), hergestellt wie unter B) beschrieben, in PBS pH 7,2 (Phosphate buffered saline) hinzugefügt und bei 37 °C inkubiert.

Danach werden 50 µl des Gemisches auf 1 cm² des flächigen immunreaktiven Trägermaterials gegeben. Man inkubiert weitere 5 Minuten bei 37 °C und zentriguiert 1 Minute mit einer Eppendorf-Zentrifuge.

Die Enzymaktivität der Digoxin-Binder-Komplexe in der freien Phase kann nunmehr kinetisch durch Zusatz von Chlorphenolrot-β-D-Galactosid (Herstellung nach DE-A- 3 345 748) bei 578 nm gemessen werden.

In den Beispielen ist jeweils der Verlauf der «ansteigenden» Eichkurven aufgezeigt, wobei die Digoxin-Konzentration des unverdünnten Serums (X-Achse) gegen die gemessene optische Dichte bei 578 nm (Y-Achse) aufgetragen ist.

Alternativ kann die überschüssige, d.h. die an die Festphase gebundene Menge Binder wie oben bestimmt und gegen die jeweilige Digoxin-Konzentration aufgetragen werden, man erhält eine «fallende» Eichkurve.

Beispiel 1

Herstellung von T3-(Trijodthyronin)-Immunpräzipitat-Vliesen.

Als Antigen wurde verwendet: Polyhapten, bestehend aus Kaninchen-IgG und daran gebundenem T3 (Molverhältnis IgG:T3 = 1:3). Das Antigen wurde immunsorptiv über eine Anti-T3-Säule aufgereinigt (siehe A)).

Als Antikörper wurde verwendet: polyklonaler Antikörper, gerichtet gegen den Fc-Teil des Kaninchen-IgG's. Der Antikörper wurde immunsorptiv über eine Kaninchen-IgG-Säule aufgereinigt (siehe A)).

Als Vlies wurde verwendet: Mischvlies aus Zellwolle und Polyester (Firma Kalff, Euskirchen, Deutschland), oder aus Linters, Zellwolle, Polyamid, (Firma Binzer, Hatzfeld, Eder, Deutschland).

Antigen und Antikörper werden getrennt in 1 mM Essigsäure aufgenommen (jeweils c = 1 mg/ml), man lässt eine Stunde bei Raumtemperatur stehen. Die Lösungen werden dann im Verhältnis 1:3 (das Antigen-/Antikörper-Verhältnis entspricht dann dem des Heidelberger-Maximums) gemischt und mit dem neunfachen Volumen einer Lösung aus 250 mM Essigsäure und 5 mM Natriumacetat verdünnt.

Das Vlies wird durch die Tränklösung gezogen, abgequetscht und 60 Minuten bei 30° im Umlufttrockenschrank getrocknet. Man wäscht eine Stunde absteigend chromatographisch mit PBS-Puffer pH 6,0 und trocknet 30 Minuten bei 30 °C im Umlufttrockenschrank.

Das Verhältnis aus Messbereich (Standard 5 ng T3/ml – Standard 0) zu Standard 0 beträgt 11,8 (siehe Tabelle 1).

Tabelle 1
Vergleich T3-Immunpräzipitat-Vliese, hergestellt nach unterschiedlichen Verfahren

| Verfahren | Beispiel | Messbereich/ Standard 0 |
|---|---|---|
| hom. 2-Schritt | 2 | 12,0 |
| hom. 1-Schritt | 1 | 11,8 |

Tabelle 1 (Fortsetzung)
Vergleich T3-Immunpräzipitat-Vliese, hergestellt
nach unterschiedlichen Verfahren

| Verfahren | Beispiel | Messbereich/Standard 0 |
|---|---|---|
| heterog. 2-Schritt | analog 4 | 1,6 |
| Suspension | analog 5 | 0,19 |

Beispiel 2
Herstellung von T3-Immunpräzipitat-Vliesen.
(Homogenes 2-Schritt-Verfahren)
Antigen, Antikörper und Vliese sind die gleichen, wie im Beispiel 1. Das Vlies wird mit 50 mM NaCl getränkt und anschliessend 30 Minuten bei 50 °C im Umlufttrockenschrank getrocknet.

Antigen und Antikörper werden getrennt in 1 mM Essigsäure aufgenommen (jeweils c = 1 mg/ml), man lässt eine Stunde bei Raumtemperatur stehen. Die beiden Lösungen werden im Verhältnis 1:3 gemischt, und mit dem neunfachen Volumen an 50 mM Essigsäure verdünnt.
Die Lösung wird auf das Vlies dosiert (Flüssigkeitssättigung des Vlieses) und das imprägnierte Vlies wird 60 Minuten bei 30 °C im Umlufttrokkenschrank getrocknet.
Man wäscht eine Stunde absteigend chromatographisch mit PBS-Puffer pH 6,0, und trocknet 30 Minuten bei 30 °C im Umlufttrockenschrank.
Das Verhältnis aus Messbereich (Standard 5 ng T3/ml – Standard 0) zu Standard 0 beträgt 12,0 (siehe Tabelle 1).

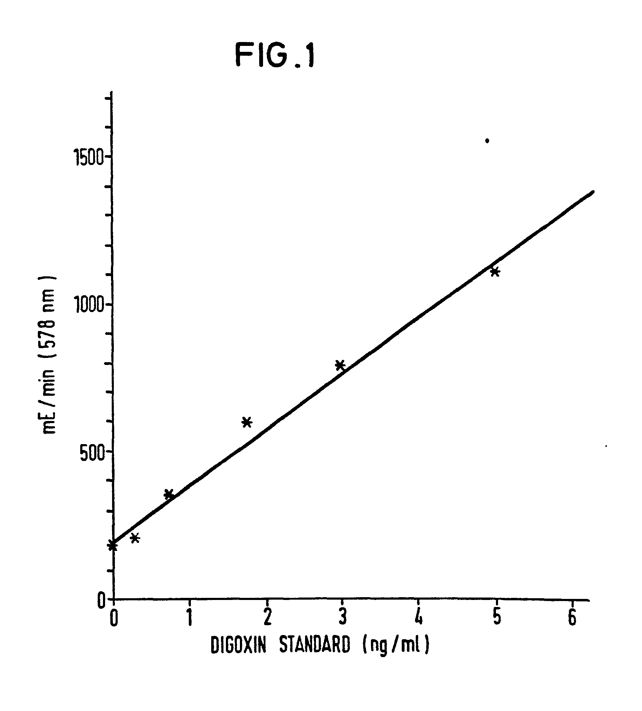

Beispiel 3
Herstellung von Digoxin-Immunpräzipitat-Vliesen. (Homogenes 1-Schritt-Verfahren)
Als Antigen wurde verwendet: Polyhapten, bestehend aus Kaninchen-IgG und daran gebundenem Digoxin (IgG: Digoxin 1:3 bis 1:4). Das Antigen wurde immunsorptiv über eine Anti-K-Fcγ-Säule aufgereinigt (siehe A)).
Als Antikörper wurde der gleiche polyklonale Antikörper eingesetzt wie im Beispiel 1.
Als Vlies wurde ein Mischvlies aus Zellwolle und Polyester verwendet (Firma Kalff, Euskirchen, Deutschland).
Antigen und Antikörper werden getrennt in 100 mM Essigsäure aufgenommen, und zwar in den Konzentrationen 1 mg/ml bzw. 3,5 mg/ml und die beiden Lösungen im Verhältnis 1:1 gemischt. Die Tränklösung wird dann mit dem vierfachen Volumen an 200 mM Natrium-citrat-Puffer, pH 3,0, verdünnt.
Das Vlies wird durch diese Tränklösung gezogen, abgequetscht, mit Umluft 10 Minuten bei 75 °C getrocknet und wie im Beispiel 1 nachgewaschen und wie zuvor getrocknet).
Mit so hergestellten Digoxin-Immunpräzipitat-Vliesen wurde die in Fig. 1 gezeigte Eichkurve erhalten. Verwendet wurden die Standards 0, 0,3, 0,75, 1,75, 3, 5 ng Digoxin/ml. Das Verhältnis von Messbereich (Standard 5 ng Digoxin/ml – Standard 0) zu Standard 0 beträgt 4,9.

Beispiel 3a
Herstellung von Digoxin-Immunpräzipitat-Vliesen (ohne Waschschritt)
Antigen (200 mg/l) und Antikörper (700 mg/l) werden getrennt in 50 mM Essigsäure aufgenommen. Nach 15 bis 30 Minuten bei Raumtemperatur werden beide Lösungen im Verhältnis 1/1 gemischt. In einem nachfolgenden Schritt wird diese Lösung mit dem gleichen Volumen an 50 mM Essigsäure plus 40 mM NaCl verdünnt. Damit werden Vliese imprägniert, 10 Minuten bei 75 °C getrocknet.
Die Vliese werden direkt in den Test eingesetzt. Die damit erhaltene Eichkurve entspricht der von Fig. 1.

Beispiel 4 (Vergleich)
Herstellung von Digoxin-Immunpräzipitat-Vliesen. (Heterogenes 2-Schritt-Verfahren)
Antigen, Antikörper und Vlies sind die gleichen wie im Beispiel 3.

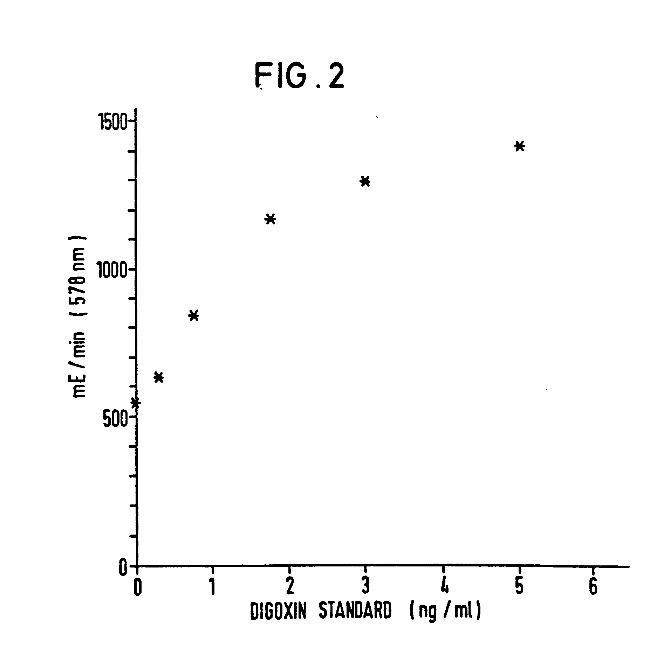

Antigen und Antikörper getrennt, werden in 1 mM Essigsäure aufgenommen (c = 5 mg/ml bzw. 17,5 mg/ml). Man lässt 30 Minuten bei Raumtemperatur stehen, dann wird die Antigen-Lösung mit dem 50fachen Volumen PBS-Puffer pH 6,0 verdünnt und auf das Vlies getränkt, man trocknet 10 Minuten bei 75 °C im Umlufttrockenschrank. Das trockene Vlies wird anschliessend mit der Antikörper-Lösung, die mit obigem Puffer 50fach verdünnt wurde, getränkt und man trocknet 10 Minuten bei 75 °C. Nach Waschen des Vlieses mit dem gleichen Puffer, wird 10 Minuten bei 75 °C getrocknet.
Man erhält die in Fig. 2 gezeigte gekrümmte Eichkurve, das Verhältnis aus Messbereich (Standard 5 ng Digoxin/ml – Standard 0) zu Standard 0 beträgt 1,6.
(Alternativ kann zunächst der Antikörper und dann das Antigen getränkt werden).

Beispiel 5 (Vergleich)
Herstellung von Digoxin-Immunpräzipitat-Vliesen. (Suspensionsverfahren)
Antigen, Antikörper und Vlies sind die gleichen wie im Beispiel 3. Antigen und Antikörper werden je für sich in 100 mM Essigsäure aufgenommen (c = 1 mg/ml bzw. c = 3,5 mg/ml), man lässt 15 Minuten bei Raumtemperatur stehen. Ein Teil Antigenlösung, ein Teil Antikörperlösung und 1,5 Teile 100 mM Kaliumphosphat-Puffer, pH 7,6, werden gemischt; man lässt eine Stunde bei Raumtemperatur stehen. Die gebildete Suspension wird abzentrifugiert, der Überstand verworfen und der Niederschlag im 9fachen Volumen PBS-Puffer pH 6,0, resuspendiert (homogenisiert). Die Suspension wird so auf das Vlies getränkt, dass das Vlies mit Flüssigkeit gesättigt ist. Danach wird 10 Minuten bei 75 °C im Umlufttrokkenschrank getrocknet. Nach einstündigem Waschen mit PBS-Puffer pH 6,0, wird erneut 10 Minuten bei 75 °C getrocknet.

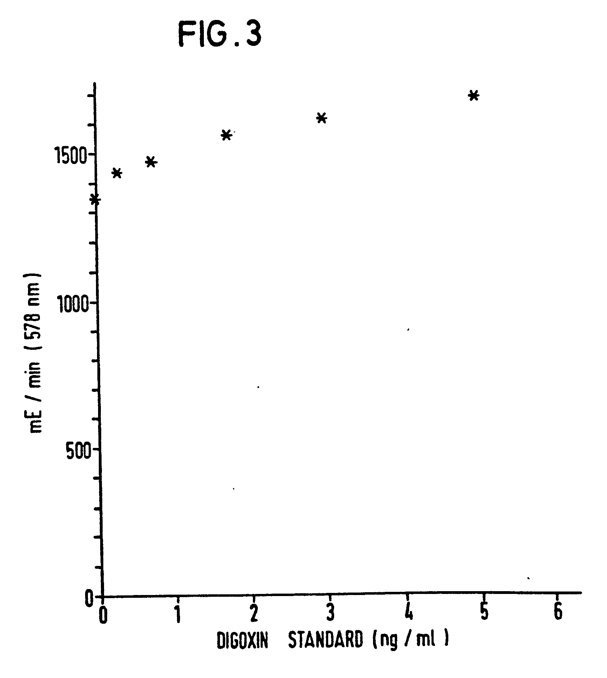

Man erhält eine gekrümmte Eichkurve (siehe Fig. 3); das Verhältnis aus Messbereich (Standard 5 ng Digoxin/ml – Standard 0) zu Standard 0 beträgt 0,19.

## Beispiel 6

Herstellung von Diphenylhydantoin (DPH) Immunpräzipitat-Vliesen (Homogenes 1-Schritt-Verfahren mit Präzipitat-Waschungen)

Als Antigen wurde verwendet: Polyhapten, bestehend aus Kaninchen-IgG und daran gebundenem Diphenylhydantoin.

Als Antikörper wurde verwendet: polyklonaler Antikörper, gerichtet gegen den Fc-Teil des Kaninchen-IgGs.

Als Vlies wurde ein Mischvlies aus Zellwolle und Polyester verwendet (Firma Kalff, Euskirchen, Deutschland).

Antigen und Antikörper (jeweils nicht immunsorptiv aufgereinigt) werden je für sich in 100 mM Essigsäure aufgenommen (c = 1 mg/ml bzw. c = 20 mg/ml); man lässt 15 Minuten bei Raumtemperatur stehen.

Ein Teil Antigen-Lösung. zwei Teile Antikörper-Lösung und zwei Teile 100 mM Kaliumphosphat-Puffer, pH 7,6, werden gemischt, man lässt die Suspension eine Stunde bei Raumtemperatur stehen. Das Präzipitat wird abzentrifugiert, der Überstand wird verworfen und das Präzipitat wird im neunfachen Volumen 100 mM Kaliumphosphat-Puffer resuspendiert. Dieser Zentrifugations-/Waschschritt wird dreimal wiederholt, dann wird das Präzipitat auf die gleiche Art noch zweimal mit destilliertem Wasser salzfrei gewaschen.

Das Präzipitat wird in 100 mM Essigsäure aufgenommen (c = 5 mg/ml); man lässt die klare Antigen-/Antikörper-Lösung 15 Minuten bei Raumtemperatur stehen und verdünnt dann mit dem vierfachen Volumen an 200 mM Natriumcitrat-Puffer, pH 3,0.

Das Vlies wird durch diese Tränklösung gezogen, abgequetscht, 10 Minuten bei 75 °C getrocknet und wie im Beispiel 1 nachgewaschen und wie zuvor getrocknet.

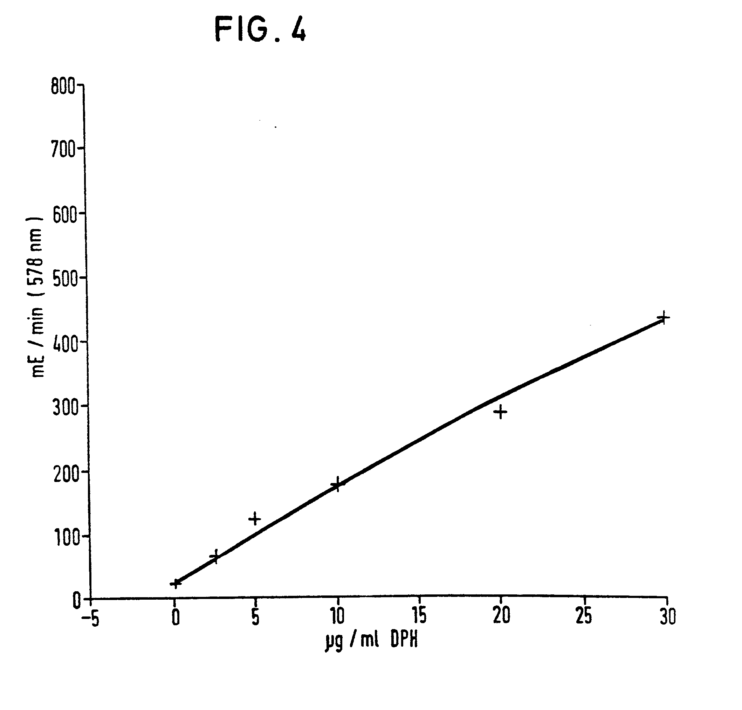

Mit so hergestellten DPH-Immunpräzipitat-Vliesen wurde die in Fig. 4 gezeigte Eichkurve erhalten. Verwendet wurden die Standards 0, 2,5, 5, 10, 20, 30 μg DPH/ml. Das Verhältnis von Messbereich (Standard 30 μg DPH/ml – Standard 0) zu Standard 0 beträgt 18,2.

## Beispiel 7

Herstellung von TSH-Immunpräzipitat-Vliesen. (Homogenes 1-Schritt-Verfahren mit Präzipitat-Waschung)

Als Antigen wurde verwendet: monoklonaler Antikörper, gerichtet gegen human-TSH. Der Antikörper (aus Ascites) wurde durch Ammonsulfat-Fällung und Chromatographie an DEAE-Cellulose aufgereinigt.

Als Antikörper wurde verwendet: polyklonaler Antikörper, gerichtet gegen den Fc-Teil des Maus-IgG. Der Antiköprer wurde durch Ammonsulfat-Fällung und Chromatographie an DEAE-Cellulose aufgereinigt.

Als Vlies wurde ein Mischvlies aus Zellwolle und Polyester verwendet (Firma Kalff, Euskirchen, Deutschland).

Antigen und Antikörper werden jeweils in 100 mM Kaliumphosphat-Puffer, pH 6,0, 0,9% NaCl, aufgenommen (c = 1 mg/ml bzw. c = 17 mg/ml). Die beiden Lösungen werden gemischt, man lässt die Suspension 30 Minuten bei Raumtemperatur stehen. Das Präzipitat wird abzentrifugiert, der Überstand wird verworfen, und das Präzipitat wird im neunfachen Volumen des obigen Puffers, resuspendiert. Dieser Zentrifugations-/Waschschritt wird fünfmal wiederholt, dann wird das Präzipitat auf die gleiche Art noch zweimal mit destilliertem Wasser salzfrei gewaschen.

Das Präzipitat wird in 50 mM Essigsäure aufgenommen (c = 5 mg/ml), man lässt die klare Antigen-/Antikörper-Lösung 15 Minuten bei Raumtemperatur stehen und verdünnt dann mit dem vierfachen Volumen an 20 mM Natriumacetat-Lösung.

Das Vlies wird durch diese Tränklösung gezogen, abgequetscht und dann 30 Minuten bei 37 °C im Umlufttrockenschrank getrocknet. Man wäscht eine Stunde absteigend chromatographisch mit Phosphatpuffer, pH 6,5 und trocknet 30 Minuten bei 37 °C.

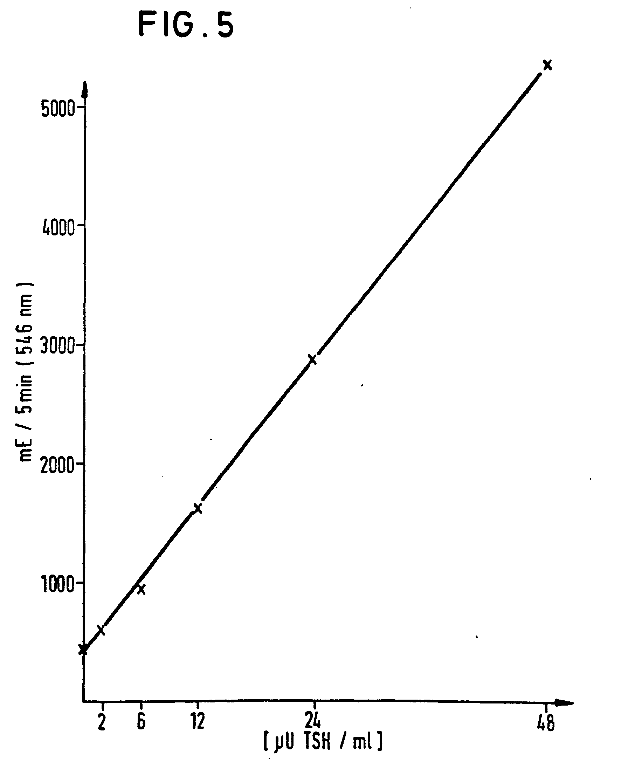

Mit so hergestellten TSH-Immunpräzipitat-Vliesen wurde die in Fig. 5 gezeigte Eichkurve erhalten; verwendet werden die Standards 0, 2, 6, 12, 24, 48 μU TSH/ml.

## Beispiel 8

Herstellung von TSH-Immunpräzipitat-Vliesen. (Homogenes 2-Schritt-Verfahren mit Präzipitat-Waschung)

Eingesetzt wurde das gewaschene Präzipitat aus Beispiel 7.

Es wurde das gleiche Vlies wie im Beispiel 7 verwendet. Das trockene Vlies wurde anschliessend mit 50 mM NaCl getränkt und getrocknet (10 Minuten 75 °C).

Das Präzipitat wurde in wenig 500 mM Essigsäure aufgenommen (gerade soviel, dass sich das Präzipitat löst), dann wird mit 5 mM Essigsäure auf c = 1 mg/ml verdünnt. Die Lösung wird auf das Vlies dosiert (Flüssigkeitssättigung des Vlieses). Man trocknet 30 Minuten bei 30 °C im Umlufttrockenschrank. Dann wird das Vlies chromatographisch mit Natriumacetat und anschliessend mit Phosphatpuffer, pH 6,5 gewaschen und bei 37 °C getrocknet.

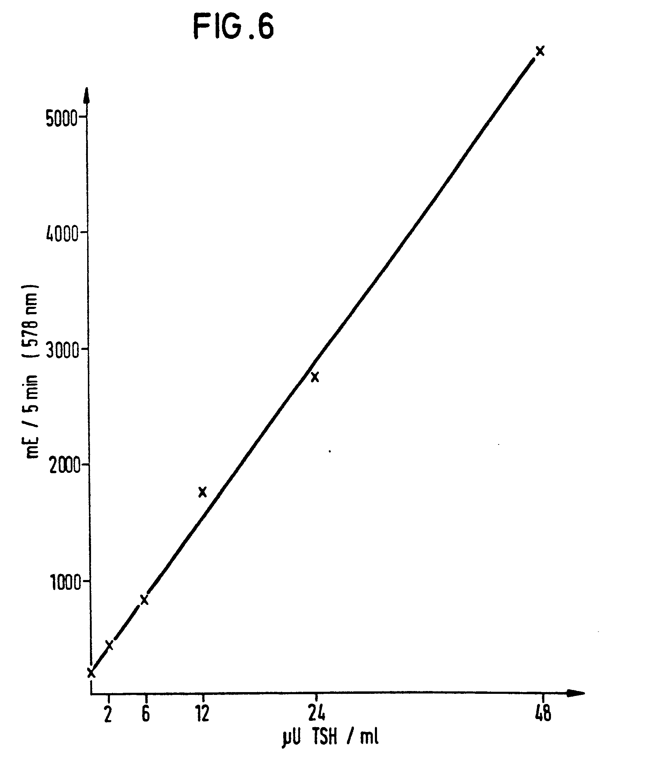

Mit so hergestellten TSH-Immunpräzipitat-Vliesen wurde die in Fig. 6 gezeigte Eichkurve erhalten, verwendet werden die Standards 0, 2, 6, 12, 24, 48 IU TSH/ml.

## Beispiel 9

Vergleich der Stabilitäten der Tränklösungen

Antigen und Antikörper sind die gleichen wie im Beispiel 1. Antigen und Antikörper werden jeweils in einer Lösung aus dem Hemmstoff, 0,1 M

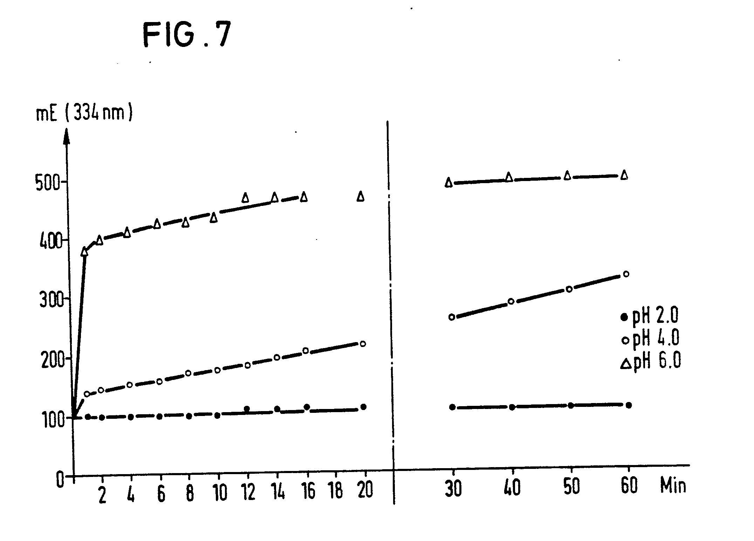

NaCl und 0,5% RSA separat gelöst. Die beiden Lösungen werden mit 0,2 N HCl (Essigsäure und Propionsäure liefern vergleichbare Ergebnisse) auf pH 6,0, pH 4,0 bzw. pH 2,0 eingestellt. Dann werden die Lösungen von Antigen und Antikörper vom jeweils gleichen pH gemischt, und der zeitliche Verlauf der Präzipitatbildung verfolgt. Fig. 7 zeigt, dass bei pH 6,0 sofort nach Mischung von Antigen und Antikörper Immunpräzipitat ausfällt, bei pH 4,0 kommt es zu geringer, nach 1 Stunde noch nicht abgeschlossener und bei pH 2,0 nicht mehr zur Präzipitatbildung.

Beispiel 10

Herstellung von T4-(Tetrajodthyronin)-Immunpräzipitat-Vliesen (ohne Waschschritt

Als Antigen wurde verwendet: Polyhapten, bestehend aus Kaninchen IgG und daran gebundenem T4 (Molverhältnis IgG:T4 = 1:3). Das Antigen wurde immunsorptiv über eine Anti-T4-Säule aufgereinigt (siehe A) ).

Als Antikörper wurde verwendet: polyklonaler Antikörper, gerichtet gegen den Fc-Teil des Kaninchen-IgG's. Der Antikörper wurde immunsorptiv über eine Kaninchen-IgG-Säule aufgereinigt (siehe A)).

Als Vlies wurde verwendet: Mischvlies aus Zellwolle und Polyester (Firma Kalff, Euskirchen, Deutschland), oder aus Linters, Zellwolle, Polyamid, (Firma Binzer, Hatzfeld, Eder, Deutschland).

Herstellung der Matrix:

Antigen (400 mg/l) und Antikörper (1400 mg/l) werden getrennt in 50 mM Essigsäure plus 0,5% NaCl aufgenommen. Nach 15 bis 30 Minuten werden beide Lösungen im Verhältnis 1/1 gemischt und damit Vliese imprägniert. Nach Trocknen (10 Minuten, 75 °C) werden die Vliese mit PBS imprägniert und getrocknet (10 Minuten, 75 °C).

Beispiel 10a

Herstellung von T4-Immunpräzipitat-Vliesen (mit Waschschritt)

Antigen (2 mg/ml) und Antikörper (7 mg/ml) werden getrennt in destilliertem Wasser gelöst. Beide Lösungen werden mit 50 mM $Na_3PO_4$ im Verhältnis 1 + 4 verdünnt. Gleiche Volumina der verdünnten Lösungen werden gemischt. Damit werden Vliese imprägniert. Die Vliese werden 10 Minuten bei 75 °C im Umlufttrockenschrank getrocknet. Anschliessend wird absteigend chromatographisch mit 10 mM Phosphat-Puffer gewaschen, bis das Eluat einen pH-Wert von 5,0 aufweist.

Nach dem Waschen wird getrocknet.

Beispiel 10b

Herstellung von $T_4$-Immunpräzipitat-Vliesen (ohne Waschschritt)

Antigen (2 mg/ml) und Antikörper (7 mg/ml) werden getrennt in destilliertem Wasser aufgelöst. Beide Lösungen werden mit 50 mM $NH_4OH$ im Verhältnis 1 + 4 verdünnt. Gleiche Volumina der verdünnten Lösungen werden gemischt. Damit werden Vliese imprägniert. Die Vliese werden 10 Minuten bei 75 °C getrocknet und direkt in den Test eingesetzt.

Die mit den Vliesen der Beispiele 10, 10a und 10b erhaltenen Eichkurven entsprechen den Eichkurven der vorhergehenden Beispiele.

**Patentansprüche**

1. Verfahren zur Herstellung eines immunreaktiven, porösen Trägermaterials durch Aufbringung von je einer Lösung eines ersten Partners einer Immunreaktion und eines mit diesem präzipitierenden zweiten Partners einer Immunreaktion, Inkubation des mit den Lösungen getränkten Trägermaterials zur Immunpräzipitation, gegebenenfalls Waschen und anschliessend Trocknen des imprägnierten Trägermaterials, dadurch gekennzeichnet, dass man eine Lösung beider Partner der Immunreaktion herstellt, welche einen Hemmstoff für die Immunpräzipitation enthält, das Trägermaterial mit dieser Lösung imprägniert und dann die Immunpräzipitation durch Entfernen des Hemmstoffes oder Aufhebung der Hemmwirkung auslöst.

2. Verfahren nach Anspruch 1, dadurch gekennzeichnet, dass man als Partner der Immunreaktion ein Protein und einen gegen dieses Protein gerichteten Antikörper oder ein Fragment davon verwendet.

3. Verfahren nach Anspruch 2, dadurch gekennzeichnet, dass man ein Protein verwendet, an das ein Hapten oder Antigen gekoppelt ist.

4. Verfahren nach Anspruch 2 oder 3, dadurch gekennzeichnet, dass man einen gegen das Protein gerichteten Antikörper oder ein Fragment davon verwendet, an den ein Hapten oder Antigen gekoppelt ist.

5. Verfahren nach Anspruch 2 bis 4, dadurch gekennzeichnet, dass man als Protein einen spezifischen Antikörper verwendet.

6. Verfahren nach einem der vorhergehenden Ansprüche, dadurch gekennzeichnet, dass man als Hemmstoff eine Säure oder eine Base verwendet.

7. Verfahren nach Anspruch 6, dadurch gekennzeichnet, dass man eine flüchtige Säure oder Base verwendet und diese nach der Imprägnierung des Trägermaterials abdampft.

8. Verfahren nach Anspruch 6, dadurch gekennzeichnet, dass man als Hemmstoff eine nicht flüchtige Säure oder Base verwendet und ein Trägermaterial einsetzt, welches mit einem Salz einer flüchtigen Säure bzw. Base imprägniert ist.

9. Verfahren nach einem der Ansprüche 1 bis 5, dadurch gekennzeichnet, dass man als Hemmstoff Glycerin oder Harnstoff einsetzt und diese nach Imprägnierung des Trägermaterials mit einem Gemisch aus mindestens einem organischen Lösungsmittel und Wasser extrahiert.

10. Verfahren nach einem der Ansprüche 1 bis 5, dadurch gekennzeichnet, dass man als Hemmstoff ein Metallsalz verwendet und dessen Hemmwirkung im Trägermaterial durch Komplexbildner aufhebt.

11. Verfahren nach einem der Ansprüche 1 bis 5, dadurch gekennzeichnet, dass man als Hemmstoff ein chaotropes Ion der Hofmeister-Serie verwendet.

12. Verfahren nach Anspruch 11, dadurch gekennzeichnet, dass man als Hemmstoff ein Thiocyanat verwendet.

13. Verfahren nach Anspruch 11, dadurch gekennzeichnet, dass man als Hemmstoff ein Alkalijodid verwendet.

14. Verfahren nach einem der vorhergehenden Ansprüche, dadurch gekennzeichnet, dass man die Partner der Immunreaktion im Verhältnis des Heidelberger-Maximums einsetzt.

15. Verfahren nach einem der vorhergehenden Ansprüche, dadurch gekennzeichnet, dass man wenigstens einen Partner der Immunreaktion in ungereinigter Form einsetzt.

16. Verfahren nach einem der vorhergehenden Ansprüche, dadurch gekennzeichnet, dass man aus den Partnern der Immunreaktion zunächst ein Suspensionspräzipitat herstellt, das nach Waschen durch Zusatz eines Hemmstoffes gelöst und anschliessend zur Tränkung des porösen Trägers eingesetzt wird.

**Revendications**

1. Procédé de préparation d'une matière pour support immunoréactive, poreuse, par application d'une solution d'un premier partenaire d'une immunoréaction et d'un second partenaire d'une immunoréaction précipitant avec celui-ci, incubation de la matière pour support imprégnée des solutions pour l'immunoprécipitation, le cas échéant lavage puis séchage de la matière pour support imprégnée, caractérisé en ce qu'on prépare une solution des deux partenaires de l'immunoréaction qui contient une substance inhibitrice pour l'immunoprécipitation, en ce qu'on imprègne la matière pour support de cette solution, puis en ce qu'on déclenche l'immunoprécipitation par élimination de la substance inhibitrice ou suppression de l'action inhibitrice.

2. Procédé suivant la revendication 1, caractérisé en ce qu'on utilise comme partenaire de l'immunoréaction une protéine et un anticorps dirigé contre cette protéine ou un fragment de celui-ci.

3. Procédé suivant la revendication 2, caractérisé en ce qu'on utilise une protéine à laquelle est couplé un haptène ou un antigène.

4. Procédé suivant les revendications 2 ou 3, caractérisé en ce qu'on utilise un anticorps dirigé contre la protéine ou un fragment de celui-ci, sur lequel est couplé un haptène ou un antigène.

5. Procédé suivant les revendications 2 à 4, caractérisé en ce qu'on utilise comme protéine un anticorps spécifique.

6. Procédé suivant l'une des revendications précédentes, caractérisé en ce qu'on utilise comme substance inhibitrice un acide ou une base.

7. Procédé suivant la revendication 6, caractérisé en ce qu'on utilise un acide ou un base volatils et en ce qu'on le fait évaporer après l'imprégnation de la matière pour support.

8. Procédé suivant la revendication 6, caractérisé en ce qu'on utilise comme substance inhibitrice un acide ou une base non volatils et une matière pour support qui est imprégnée d'un sel d'un acide ou d'une base volatils.

9. Procédé suivant l'une des revendications 1 à 5, caractérisé en ce que l'on utilise comme substance inhibitrice du glycérol ou de l'urée et en ce qu'on l'extrait après imprégnation de la matière pour support avec un mélange d'au moins un solvant organique et d'eau.

10. Procédé suivant l'une des revendications 1 à 5, caractérisé en ce qu'on utilise comme substance inhibitrice un sel métallique et en ce qu'on fait cesser l'action inhibitrice de celui-ci dans la matière pour support au moyen de complexants.

11. Procédé suivant l'une des revendications 1 à 5, caractérisé en ce qu'on utilise comme substance inhibitrice un ion chaotrope de la série d'Hofmeister.

12. Procédé suivant la revendication 11, caractérisé en ce qu'on utilise comme substance inhibitrice un thiocyanate.

13. Procédé suivant la revendication 11, caractérisé en ce qu'on utilise comme substance inhibitrice un iodure alcalin.

14. Procédé suivant l'une des revendications précédentes, caractérisé en ce qu'on utilise les partenaires de l'immunoréaction dans le rapport du maximum d'Heidelberger.

15. Procédé suivant l'une des revendications précédentes, caractérisé en ce qu'on utilise au moins un partenaire de l'immunoréaction sous forme non purifiée.

16. Procédé suivant l'une des revendications précédentes, caractérisé en ce qu'on prépare d'abord, à partir des partenaires de l'immunoréaction, un précipité en suspension qui est dissous après lavage par addition d'une substance inhibitrice, puis est utilisé pour l'imprégnation du support poreux.

**Claims**

1. Process for the preparation of an immunoreactive, porous carrier material by application of, in each case, a solution of a first reaction component of an immuno-reaction and of a second component of an immuno-reaction precipitating therewith, incubation of the carrier material impregnated with the solutions for the immuno-precipitation, optional washing and subsequent drying of the impregnated carrier material, characterised in that one prepares a solution of both components of the immuno-reaction, which contains an inhibitor for the immuns precipitation, impregnates the carrier material with this solution and then initiates the immuno-precipitation by removal of the inhibitor or removal of the inhibiting action.

2. Process according to claim 1, characterised in that, as components of the immuno-reaction, one uses a protein and an antibody or a fragment thereof directed against this protein.

3. Process according to claim 2, characterised in that one uses a protein to which a hapten or antigen is coupled.

4. Process according to claim 2 or 3, characterised in that one uses an antibody or a fragment thereof directed against the protein to which a hapten or antigen is coupled.

5. Process according to claim 2 to 4, characterised in that one uses a specific antibody as protein.

6. Process according to one of the preceding claims, characterised in that one uses an acid or a base as inhibitor.

7. Process according to claim 6, characterised in that one uses a volatile acid or base and evaporates this off after the impregnation of the carrier material.

8. Process according to claim 6, characterised in that one uses a non-volatile acid or base as inhibitor and employs a carrier material which is impregnated with a salt of a volatile acid or base.

9. Process according to one of claims 1 to 5, characterised in that, as inhibitor, one uses glycerol or urea and, after impregnation of the carrier material, extracts with a mixture of at least one organic solvent and water.

10. Process according to one of claims 1 to 5, characterised in that, as inhibitor, one uses a metal salt and removes its inhibiting action in the carrier material by means of complex formers.

11. Process according to one of claims 1 to 5, characterised in that, as inhibitor, one uses a chaotropic ion of the Hofmeister series.

12. Process according to claim 11, characterised in that one uses a thiocyanate as inhibitor.

13. Process according to claim 11, characterised in that one uses an alkali metal iodide as inhibitor.

14. Process according to one of the preceding claims, characterised in that one uses the components of the immuno-reaction in the ratio of the Heidelberger maximum.

15. Process according to one of the preceding claims, characterised in that one uses at least one component of the immuno-reaction in non-purified form.

16. Process according to one of the preceding claims, characterised in that from the components of the immunoreaction one first prepares a suspension precipitate which, after washing, is dissolved by the addition of an inhibitor and is subsequently used for the impregnation of the porous carrier.

1/7

# FIG.1

2/7

FIG.2

3/7

## FIG.3

4/7

# FIG. 4

5/7

FIG.5

6/7

## FIG.6

FIG.7